# EUROPEAN PATENT SPECIFICATION

(11) **EP 2 938 300 B1**
(45) Date of publication and mention of the grant of the patent: **02.05.2018**
(21) Application number: 13867379.3
(22) Date of filing: 20.12.2013
(51) Int. Cl.: A61F 5/01, A61F 5/042, A61F 5/04, A61B 17/80, A61B 17/70

(54) **ORTHOPEDIC BONE PLATE AND LOCKING TAB APPARATUS**
ORTHOPÄDISCHE KNOCHENPLATTE UND VERRIEGELUNGSLASCHE
PLAQUE VISSÉE ORTHOPÉDIQUE ET APPAREIL À LANGUETTE DE VERROUILLAGE

(30) Priority: 28.12.2012 US 201261746901 P
(43) Date of publication of application: 04.11.2015
(73) Proprietor: Paragon 28, Inc., Englewood, Colorado 80112 (US)
(72) Inventor: DACOSTA, Albert, Englewood, Colorado 80112 (US); SANGIOVANNI, Thomas, Miami, Florida 33146 (US)
(74) Representative: Buzzi, Franco
(86) International application number: PCT/US2013/077173
(87) International publication number: WO 2014/105742

(56) References cited:
- EP-A2- 1 356 778
- FR-A1- 2 785 519
- US-A- 5 662 655
- US-A1- 2003 225 409
- US-A1- 2005 085 814
- US-A1- 2007 073 298
- US-A1- 2010 152 737
- US-A1- 2011 087 229
- US-A1- 2012 184 959
- US-B1- 6 416 528

## Description

### FIELD OF THE INVENTION

The present invention relates generally to the field of orthopedics related to an orthopedic bone plate and locking tab apparatus. FR2785519A1, EP1356778A2, US2012/184959 and US2011/087229A1 disclose an orthopedic plate according to the preamble of Claim 1.

### SUMMARY OF THE INVENTION

The present invention is directed toward devices for securing an orthopedic bone plate over an osteotomy.

In one aspect of the present invention provided herein is an orthopedic bone plate including a body with a first end, a second end, and a central portion extending between the first end and the second end. The orthopedic bone plate may also include a first pair of lobes at the first end and a second pair of lobes at the second end. In addition, the orthopedic bone plate may include a securing mechanism extending away from a bottom surface of the central portion.

In another aspect of the present invention provided herein is a bone plate securing mechanism that is secured to a bone plate including a body portion with at least two attachment openings. The bone plate securing mechanism includes a base portion projecting away from the body portion of the bone plate and two opposing extension members projecting from the base portion. Disclosed is a method, which is not part of the invention, for inserting a bone plate. The method includes obtaining the bone plate. The bone plate including a body with a first end, a second end, and a central portion extending between the first end and the second end, a first pair of lobes at the first end, a second pair of lobes at the second end, and a securing mechanism projecting from a bottom surface of the central portion. The method also includes making an incision in a patient over at least one bone requiring correction. The method further includes performing an osteotomy on the at least one bone to form a first bone portion and a second bone portion. In addition, the method may include repositioning the first bone portion. Further, the method includes inserting the securing mechanism of the bone plate between the first bone portion and the second bone portion. The method may also include positioning the bone plate between the first bone portion and the second bone portion. The method may include securing the first end of the bone plate to the first bone portion. Further, the method may include moving the first bone portion and the second bone portion to a desired position to engage the securing mechanism. In addition, the method may include securing the second end of the bone plate to the second bone portion. The method may finally include closing the incision in the patient.

These, and other objects, features and advantages of this invention will become apparent from the following detailed description of the various aspects of the invention taken in conjunction with the accompanying drawings.

### BRIEF DESCRIPTION OF DRAWINGS

The accompanying drawings, which are incorporated in and constitute a part of the specification, illustrate embodiments of the invention and together with the detailed description herein, serve to explain the principles of the invention. It is emphasized that, in accordance with the standard practice in the industry, various features are not drawn to scale. In fact, the dimensions of the various features may be arbitrarily increased or reduced for clarity of discussion. The drawings are only for purposes of illustrating preferred embodiments and are not to be construed as limiting the invention.
**FIG. 1** is a lateral view of an orthopedic bone plate, in accordance with an aspect of the present invention;
**FIG. 2** is an end perspective view of the plate of FIG. 1, in accordance with an aspect of the present invention;
**FIG. 3** is a lateral perspective view of the plate of FIG. 1, in accordance with an aspect of the present invention;
**FIG. 4** is a bottom perspective view of the plate of FIG. 1, in accordance with an aspect of the present invention;
**FIG. 5** is a bottom view of the plate of FIG. 1, in accordance with an aspect of the present invention;
**FIG. 6** is a top view of the plate of FIG. 1, in accordance with an aspect of the present invention; and
**FIG. 7** depicts one embodiment of a method for inserting a bone plate.

### DETAILED DESCRIPTION FOR CARRYING OUT THE INVENTION

Generally stated, disclosed herein is an embodiment of an orthopedic bone plate as well as a securing mechanism. Further, a surgical method, which is not part of the invention, for using the orthopedic bone plate is discussed.

In this detailed description and the following claims, the words proximal, distal, anterior, posterior, medial, lateral, superior and inferior are defined by their standard usage for indicating a particular part of a bone or implant according to the relative disposition of the natural bone or directional terms of reference. For example, "proximal" means the portion of an implant nearest the torso, while "distal" indicates the portion of the implant farthest from the torso. As for directional terms, "anterior" is a direction towards the front side of the body, "posterior" means a direction towards the back side of the body, "medial" means towards the midline of the body, "lateral" is a direction towards the sides or away from the midline of the body, "superior" means a direction above and "inferior" means a direction below another object or structure.

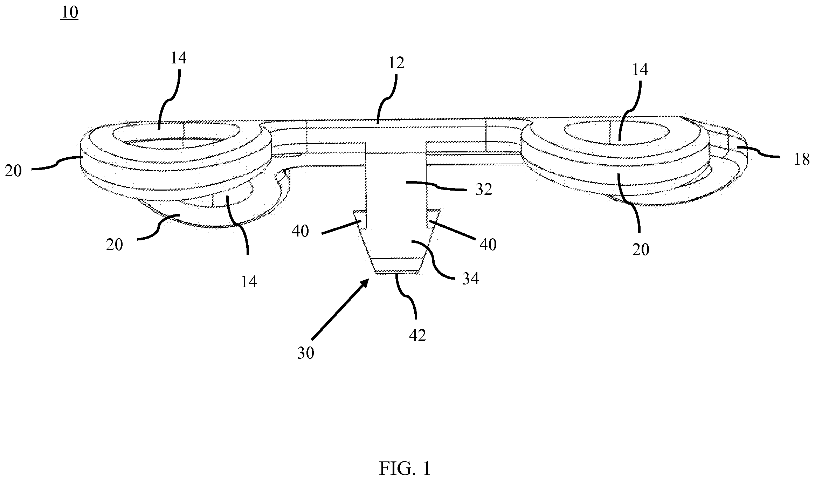

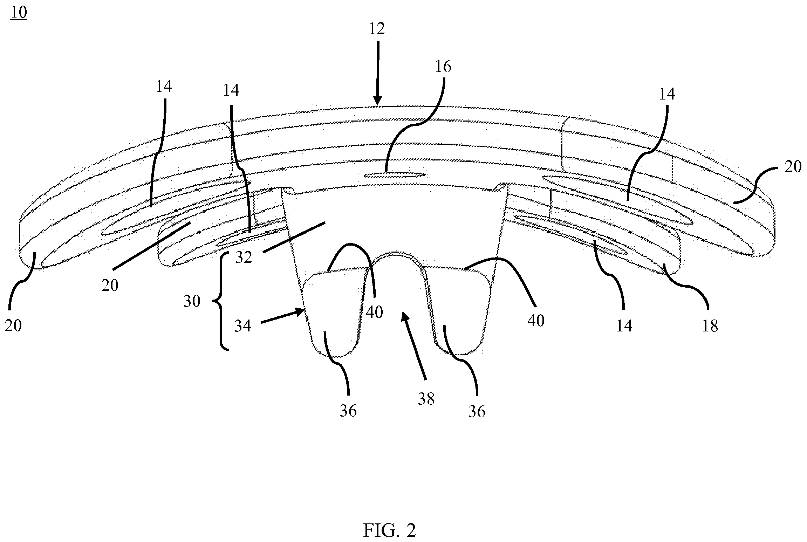

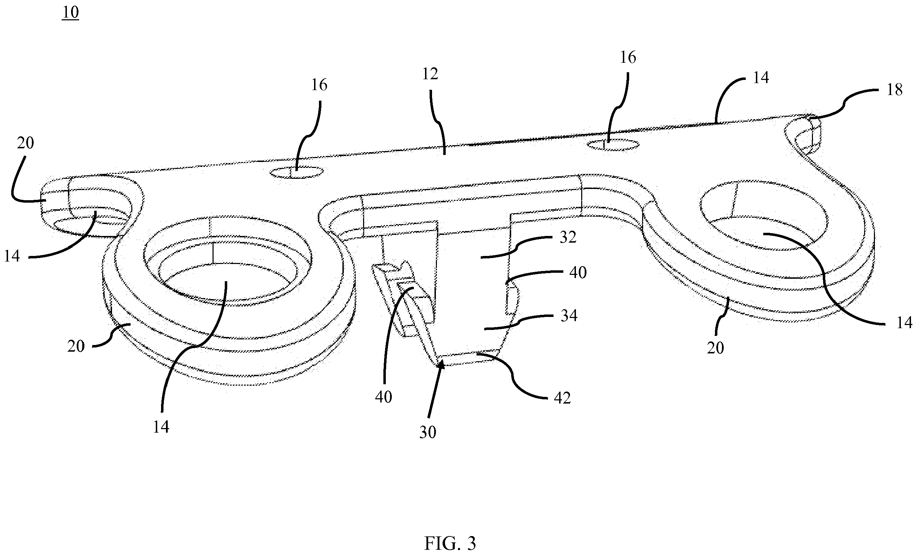

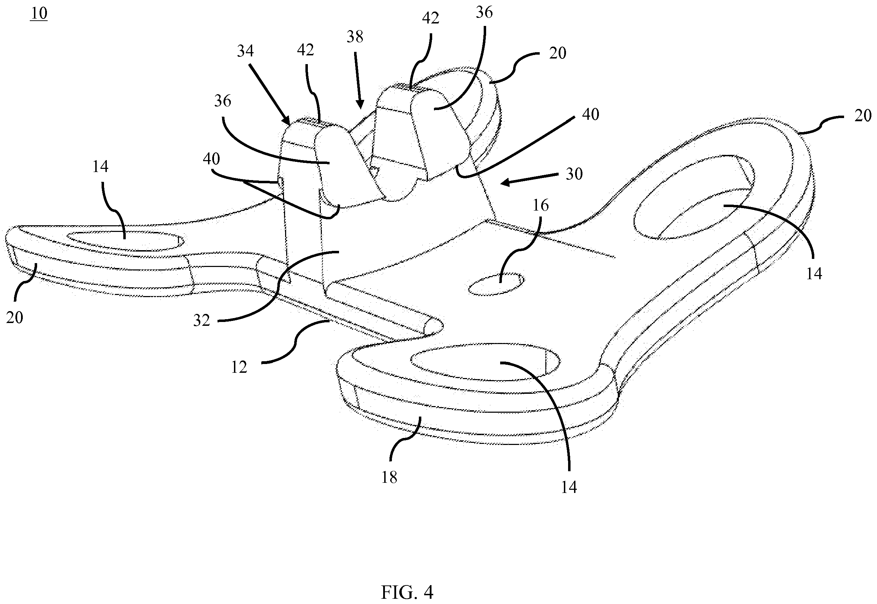

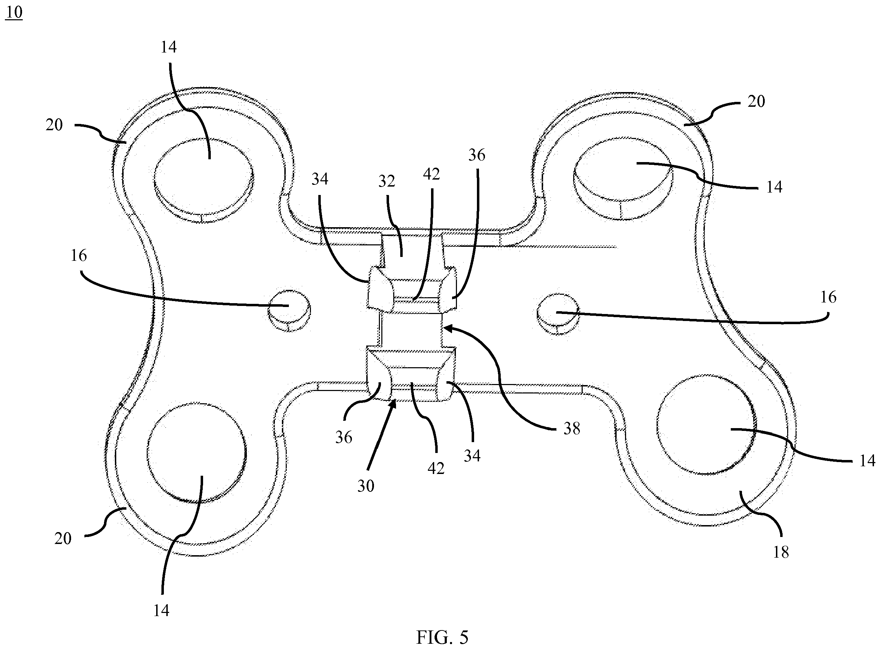

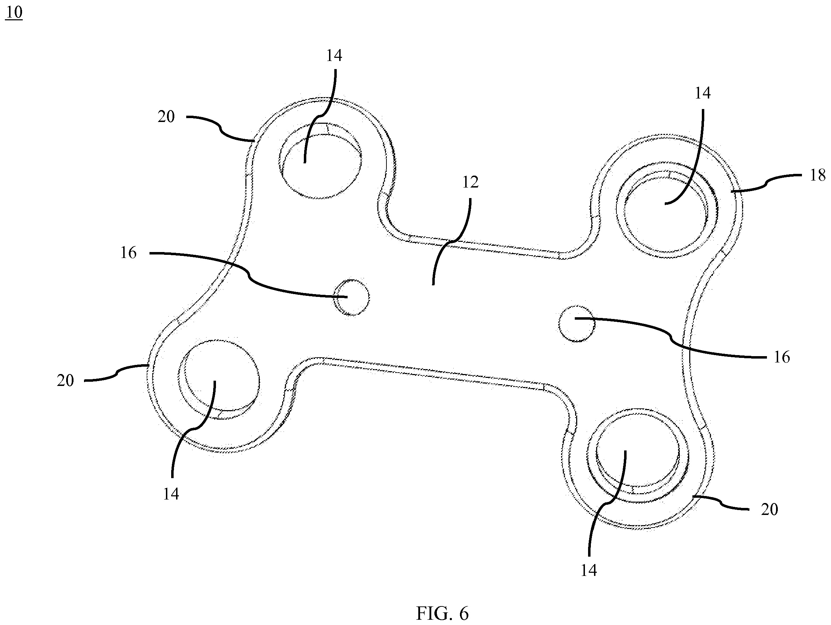

Referring to the drawings, wherein like reference numerals are used to indicate like or analogous components throughout the several views, and with particular reference to FIGS. 1-6, there is illustrated an exemplary embodiment orthopedic bone plate 10. The plate 10 may be, for example, a base opening wedge plate or BOW plate. The plate 10 may include a body 12 with one or more holes or screw holes 14, at least one opening 16, and a securing mechanism or locking mechanism 30. The body 12 may include a first end, a second end, and a central portion extending between the first end and second end. The plate 10 may have, for example, a longitudinal curvature for conforming to the bone(s) it is attached to. The plate 10 may also have, for example, a diametral curvature for conforming to the surface of the outer cortex of the bone or bones which the plate 10 is attached to, as shown in FIG. 2. For example purposes, the plate 10 is shown as being generally I-shaped (See FIG. 5) with a generally rectangular shaped body 12 and including two pairs of rounded lobes, arms, ears, tabs, or the like. However, other plate shapes are also contemplated for use and such other shapes may be necessary to address certain clinical situations.

With continued reference to FIGS. 1-6, the screw holes 14 may be located in a first pair of rounded lobes and a second pair of rounded lobes extending from body 12. For example, the first pair of lobes at the first end of the body 12 may include a first lobe 18, which is offset from a second lobe 20, while the second pair of lobes at the second end of the body 12 may have two parallel oriented second lobes 20. Thus, in the depicted embodiment there are preferably four screw holes 14 corresponding to the four lobes 18, 20. However, it is contemplated that a plurality of screw holes may be present in each lobe 18, 20. The one or more screw holes 14 may be threaded or non-threaded holes. The one or more screw holes 14 in the second pair of rounded lobes, for example, the proximal lobes, may be, for example, angled to receive a fastener, such as, a bone screw, which may be positioned to converge toward the securing mechanism 30. By orienting the fasteners in the proximal lobes toward the securing mechanism 30, the fasteners may avoid the joint cartilage that curves inward in the patient's joint. Thus, the surgeon may be able to position the plate 10 to make the osteotomy more proximal to improve healing due to the osteotomy being in a more vascular space.

The at least one opening 16 may be located along the longitudinal axis of the plate 10 in the body 12. In the depicted embodiment, for example, the plate 10 may include two openings 16 aligned along the center of the body 12 where the first and second pairs of rounded lobes extend out from the body 12. The two openings 16 may be, for example, used for inserting temporary fixation pins, olive wires, k-wires or the like during attachment of the plate 10 to the patient's bone or bones.

The securing mechanism 30, as shown in FIGS. 1-6, may include a base portion 32 extending inferior from a bone contacting surface of the central portion of the body 12. The securing mechanism 30 may also include at least two opposing extension members or wedge portions 34 projecting from the base portion 32 to an insertion end 42. The extension members 34 may include at least two wedges 36 and a cavity or intermediate cavity 38. In depicted embodiment, the extension members 34 may include, for example, two wedges 36 and a cavity 38. The at least two extension members 34 may also include, a plurality of retaining members, shoulders, or lips 40. The plurality of shoulders 40 may be positioned along a side of each of the extension members 34 or protrude away from the extension members 34 with for example one shoulder 40 in a proximal direction and a second shoulder 40 in a distal direction. The insertion end 42 is positioned at an end of each of the plurality of retaining members 40 opposite the base portion 32. The at least one wedge 36 of the wedge portion 34 may be tapered or angled from the lips 40 to the insertion end 42 as it extends inferiorly from the base 32. The securing mechanism 30 may be tapered or angled, for example, in both the proximal-distal direction and medial-lateral direction, as shown in FIGS. 1 and 2, respectively. It is also contemplated that the securing mechanism 30 may only be tapered or angled in one direction. The above described securing mechanism 30 may also be incorporated into other orthopedic bone plates which may be used to secure at least two bones together in a desired orientation after, for example, an osteotomy.

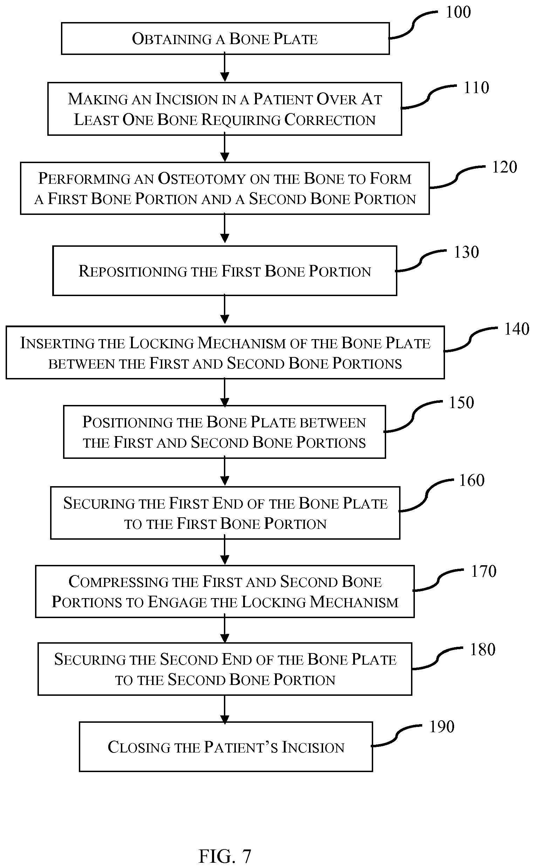

As depicted in FIG. 7, the method for insertion of the orthopedic plate, which is not part of the invention, includes, the surgeon making an incision in the patient to perform an osteotomy on the bone or bones requiring correction. During the osteotomy a first bone may be cut leaving a connection between the two portions of the first bone. The connection between the two portions of the first bone may act as a hinge. If the osteotomy was, for example, being performed on the foot bones, then the hinge may be an intact lateral cortex. Once the osteotomy is completed, the first bone may be repositioned and the plate 10 may be inserted. When the plate 10 is inserted into the patient, the securing mechanism 30 of the plate 10 may be slid into place between the portions of the bone or bones created by the osteotomy.

After the securing mechanism 30 and plate 10 are inserted into the patient the surgeon may position the plate 10 in a desired position within the osteotomy and over the bones. A temporary fixation pin, olive wire, k-wire or the like may be inserted into one of the openings 16 to hold the plate 10 in the desired position in a first portion of the bone. In the preferred method olive wires are used because the olive wires contain a stopper which holds the plate 10 down on the bone while the fasteners are inserted. Alternative temporary fixation mechanisms that include a stopper or means to hold the plate 10 to the bones are also contemplated. Then plate 10 may be fastened to the first portion of the bone using at least one fastener, such as a bone screw. In the depicted embodiment, two fasteners would be inserted into two of the openings 14 on one end of the plate 10. Once the two fasteners are inserted and the plate is secured to the bone at one end the temporary fixation pin may be removed.

The surgeon may then move or compress the bones or portions of the bone to a desired position and insert a temporary fixation pin, olive wire, k-wire or the like into the other opening 16 to hold the plate 10 in the desired position on the second portion of the bone. As the bones are moved the cortical wall on both sides of the osteotomy engages the lips 40 of the securing mechanism 30. The plate 10 may then be fastened at the second end to the second portion of the first bone using at least one fastener, such as a bone screw. In the depicted embodiment, two fasteners would be inserted into the two openings 14 on the second end of the plate 10. After the fasteners are inserted into the bone and plate 10 is secured, the temporary fixation pin may be removed. Once the fasteners are inserted into both ends of the plate 10 securing the plate 10 to the bone, the securing mechanism 30 is engaging the cortical wall on both sides of the osteotomy. The engagement of the cortical wall by the securing mechanism 30 prevents the securing mechanism 30 from backing out of the osteotomy and holds the plate 10 down on the bone. Once the plate 10 is secured to the bone, the surgeon may then close the patient's incision.

In one embodiment, as shown in FIG. 7, a method for inserting a bone plate which is not part of the present invention may include, for instance: obtaining the bone plate 100; making an incision in a patient over at least one bone requiring correction 110; performing an osteotomy on the at least one bone to form a first bone portion and a second bone portion 120; repositioning the first bone portion 130; inserting the securing mechanism of the bone plate between the first bone portion and the second bone portion 140; positioning the bone plate between the first bone portion and the second bone portion 150; securing the first end of the bone plate to the first bone portion 160; moving the first bone portion and the second bone portion to a desired position to engage the securing mechanism 170; securing the second end of the bone plate to the second bone portion 180; and closing the incision in the patient 190.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprise" (and any form of comprise, such as "comprises" and "comprising"), "have" (and any form of have, such as "has", and "having"), "include" (and any form of include, such as "includes" and "including"), and "contain" (and any form of contain, such as "contains" and "containing") are open-ended linking verbs. As a result, a method or device that "comprises," "has," "includes," or "contains" one or more steps or elements possesses those one or more steps or elements, but is not limited to possessing only those one or more steps or elements. Likewise, a step of a method or an element of a device that "comprises," "has," "includes," or "contains" one or more features possesses those one or more features, but is not limited to possessing only those one or more features. Furthermore, a device or structure that is configured in a certain way is configured in at least that way, but may also be configured in ways that are not listed.

## Claims

1. An orthopedic plate (10), comprising:
a body (12) with a first end, a second end, and a central portion extending between the first endand the second end;
a first pair of lobes (18, 20) at the first end;
a second pair of lobes (20) at the second end; and
a securing mechanism (30) extending away from a bottom surfaceof the central portion, said orthopedic plate being **characterized in that** the securing mechanism (30) comprises:
a base portion (32);
at least two opposing extension members (34) projecting from the base portion (32);
at least two retaining members (40) positioned along a side of each of the two opposing extension members (34), wherein at least one first retaining member (40) protrudes from at least one of the at least two opposing extension members (34) in a proximal direction and at least one second retaining member (40) protrudes from at least one of the at least two opposing extension members (34) in a distal direction.

2. The orthopedic plate (10) of claim 1, wherein the securing mechanism (30) further comprises:
a cavity (38) positioned between the at least two opposing extension members (34).

3. The orthopedic plate (10) of claim 1, wherein the at least two opposing extension members (34) further comprise an insertion end (42) positioned at an end of each of the at least two retaining members (40) opposite the base portion (32).

4. The orthopedic plate (10) of claim 3, wherein the at least two opposing extension members (34) are tapered from the at least two retaining members (40) to the insertion end (42) and in at least one of a proximal-distal direction and a medial-lateral direction.

5. The orthopedic plate (10) of claim 1, further comprising:
at least one hole (14) in the first end of the body (12);
at least one hole (14) in the second end of the body (12); and
at least one opening (16) in the central portion of the body (12).

6. The orthopedic plate (10) of claim 5, wherein the at least one hole (14) in the first end comprises at least one hole (14) in the first pair of lobes (18, 20) and the at least one hole (14) in the second end comprises at least one hole (14) in the second pair of lobes (20).

7. The orthopedic plate (10) of claim 6, wherein each of the lobes (20) in the second pair of lobes (20) includes at least one hole (14) and each of the at least one holes (14) is angled toward the securing mechanism (30).

8. The orthopedic plate (10) of claim 1, wherein the first pair of lobes (18, 20) includes a first lobe (18) offset from a second lobe (20) and the second pair of lobes (20) includes a first lobe (20) oriented parallel to a second lobe (20).

9. The orthopedic plate (10) of claim 1, wherein the body (12) has a longitudinal curvature from the first end to the second end and a diametral curvature from a medial side to a lateral side.

10. The orthopedic plate (10) of claim 1, wherein the two opposing extension members (34) define a cavity (38) positioned intermediate the at least two retaining members (40).

11. The orthopedic plate (10) of claim 10, wherein the two opposing extension members (34) further comprise an insertion end (42).

12. The orthopedic plate (10) of claim 11, wherein the at least two opposing extension members (34) are angled from the at least two retaining members (40) to the insertion end (42) and in at least one of a proximal-distal direction and a medial-lateral direction.

## Patentansprüche

1. Orthopädische Platte (10), umfassend:
einen Körper (12) mit einem ersten Ende, einem zweiten Ende und einem sich zwischen dem ersten Ende und dem zweiten Ende erstreckenden mittleren Abschnitt
ein erstes Paar Flügel (18, 20) am ersten Ende,
ein zweites Paar Flügel (20) am zweiten Ende und
einen Sicherungsmechanismus (30), der sich von einer Bodenfläche des mittleren Abschnitts aus von dieser weg erstreckt, wobei die orthopädische Platte **dadurch gekennzeichnet ist, dass** der Sicherungsmechanismus (30) Folgendes umfasst:
einen Sockelabschnitt (32),
mindestens zwei einander gegenüberliegende Verlängerungselemente (34), die vom Sockelabschnitt (32) abstehen,
mindestens zwei Halteelemente (40), die entlang einer Seite jedes der zwei einander gegenüberliegenden Verlängerungselemente (34) positioniert sind, wobei mindestens ein erstes Halteelement (40) von mindestens einem der mindestens zwei einander gegenüberliegenden Verlängerungselemente (34) in einer proximalen Richtung vorsteht und mindestens ein zweites Halteelement (40) von mindestens einem der mindestens zwei einander gegenüberliegenden Verlängerungselemente (34) in einer distalen Richtung vorsteht.

2. Orthopädische Platte (10) nach Anspruch 1, wobei der Sicherungsmechanismus (30) ferner Folgendes umfasst:
eine zwischen den mindestens zwei einander gegenüberliegenden Verlängerungselementen (34) positionierte Vertiefung (38).

3. Orthopädische Platte (10) nach Anspruch 1, wobei die mindestens zwei einander gegenüberliegenden Verlängerungselemente (34) ferner ein Einführungsende (42) umfassen, das an einem dem Sockelabschnitt (32) entgegengesetzten Ende jedes der mindestens zwei Halteelemente (40) positioniert ist.

4. Orthopädische Platte (10) nach Anspruch 3, wobei sich die mindestens zwei einander gegenüberliegenden Verlängerungselemente (34) von den mindestens zwei Halteelementen (40) zum Einführungsende (42) und in einer proximal-distalen Richtung und/oder einer medial-lateralen Richtung verjüngen.

5. Orthopädische Platte (10) nach Anspruch 1, ferner umfassend:
mindestens eine Öffnung (14) im ersten Ende des Körpers (12),
mindestens eine Öffnung (14) im zweiten Ende des Körpers (12) und
mindestens eine Öffnung (16) im mittleren Abschnitt des Körpers (12).

6. Orthopädische Platte (10) nach Anspruch 5, wobei die mindestens eine Öffnung (14) im ersten Ende mindestens eine Öffnung (14) im ersten Paar Flügel (18, 20) umfasst und die mindestens eine Öffnung (14) im zweiten Ende mindestens eine Öffnung (14) im zweiten Paar Flügel (20) umfasst.

7. Orthopädische Platte (10) nach Anspruch 6, wobei jeder der Flügel (20) des zweiten Paars Flügel (20) mindestens eine Öffnung (14) aufweist und jede der mindestens einen Öffnungen (14) zum Sicherungsmechanismus (30) hin gewinkelt ist.

8. Orthopädische Platte (10) nach Anspruch 1, wobei das erste Paar Flügel (18, 20) einen ersten Flügel (18) aufweist, der zu einem zweiten Flügel (20) versetzt ist, und das zweite Paar Flügel (20) einen ersten Flügel (20) aufweist, der parallel zu einem zweiten Flügel (20) ausgerichtet ist.

9. Orthopädische Platte (10) nach Anspruch 1, wobei der Körper (12) eine Längskrümmung vom ersten zum zweiten Ende und eine diametrale Krümmung von einer medialen Seite zu einer lateralen Seite besitzt.

10. Orthopädische Platte (10) nach Anspruch 1, wobei die zwei einander gegenüberliegenden Verlängerungselemente (34) eine zwischen den mindestens zwei Halteelementen (40) positionierte Vertiefung (38) definieren.

11. Orthopädische Platte (10) nach Anspruch 10, wobei die zwei einander gegenüberliegenden Verlängerungselemente (34) ferner ein Einführungsende (42) umfassen.

12. Orthopädische Platte (10) nach Anspruch 11, wobei die mindestens zwei einander gegenüberliegenden Verlängerungselemente (34) von den mindestens zwei Halteelementen (40) zum Einführungsende (42) und in einer proximal-distalen Richtung und/oder einer medial-lateralen Richtung gewinkelt sind.

## Revendications

1. Plaque orthopédique (10), comprenant :
un corps (12) ayant une première extrémité, une deuxième extrémité, et une partie centrale s'étendant entre la première extrémité et la deuxième extrémité ;
une première paire de lobes (18, 20) au niveau de la première extrémité ;
une deuxième paire de lobes (20) au niveau de la deuxième extrémité ; et
un mécanisme de fixation (30) s'étendant à l'opposé d'une surface inférieure de la partie centrale, ladite plaque orthopédique étant **caractérisée en ce que** le mécanisme de fixation (30) comprend :
une partie de base (32) ;
au moins deux éléments d'extension opposés (34) faisant saillie à partir de la partie de base (32) ;
au moins deux éléments de retenue (40) positionnés le long d'un côté de chacun des deux éléments d'extension opposés (34), où au moins un premier élément de retenue (40) fait saillie à partir d'au moins l'un parmi les au moins deux éléments d'extension opposés (34) dans une direction proximale et au moins un deuxième élément de retenue (40) fait saillie à partir d'au moins l'un parmi les au moins deux éléments d'extension opposés (34) dans une direction distale.

2. Plaque orthopédique (10) de la revendication 1, dans laquelle le mécanisme de fixation (30) comprend en outre :
une cavité (38) positionnée entre les au moins deux éléments d'extension opposés (34).

3. Plaque orthopédique (10) de la revendication 1, dans laquelle les au moins deux éléments d'extension opposés (34) comprennent en outre une extrémité d'insertion (42) positionnée au niveau d'une extrémité de chacun des au moins deux éléments de retenue (40) en face de la partie de base (32).

4. Plaque orthopédique (10) de la revendication 3, dans laquelle les au moins deux éléments d'extension opposés (34) se rétrécissent à partir des au moins deux éléments de retenue (40) vers l'extrémité d'insertion (42) et dans au moins l'une parmi une direction proximale-distale et une direction médiale-latérale.

5. Plaque orthopédique (10) de la revendication 1, comprenant en outre :
au moins un trou (14) dans la première extrémité du corps (12) ;
au moins un trou (14) dans la deuxième extrémité du corps (12) ; et
au moins une ouverture (16) dans la partie centrale du corps (12).

6. Plaque orthopédique (10) de la revendication 5, dans laquelle l'au moins un trou (14) dans la première extrémité comprend au moins un trou (14) dans la première paire de lobes (18, 20) et l'au moins un trou (14) dans la deuxième extrémité comprend au moins un trou (14) dans la deuxième paire de lobes (20).

7. Plaque orthopédique (10) de la revendication 6, dans laquelle chacun des lobes (20) dans la deuxième paire de lobes (20) comporte au moins un trou (14) et chacun de l'au moins un trou (14) est incliné vers le mécanisme de fixation (30).

8. Plaque orthopédique (10) de la revendication 1, dans laquelle la première paire de lobes (18, 20) comporte un premier lobe (18) décalé par rapport à un deuxième lobe (20) et la deuxième paire de lobes (20) comporte un premier lobe (20) orienté parallèlement à un deuxième lobe (20).

9. Plaque orthopédique (10) de la revendication 1, dans laquelle le corps (12) a une courbure longitudinale à partir de la première extrémité vers la deuxième extrémité et une courbure diamétrale à partir d'un côté médial vers un côté latéral.

10. Plaque orthopédique (10) de la revendication 1, dans laquelle les deux éléments d'extension opposés (34) définissent une cavité (38) positionnée entre les au moins deux éléments de retenue (40).

11. Plaque orthopédique (10) de la revendication 10, dans laquelle les deux éléments d'extension opposés (34) comprennent en outre une extrémité d'insertion (42).

12. Plaque orthopédique (10) de la revendication 11, dans laquelle les au moins deux éléments d'extension opposés (34) sont inclinés à partir des au moins deux éléments de retenue (40) vers l'extrémité d'insertion (42) et dans au moins l'une parmi une direction proximale-distale et une direction médiale-latérale.
